# EUROPÄISCHE PATENTSCHRIFT

(11) **EP 0 982 038 B1**
(45) Veröffentlichungstag und Bekanntmachung des Hinweises auf die Patenterteilung: **08.01.2003**
(21) Anmeldenummer: 99116089.6
(22) Anmeldetag: 17.08.1999
(51) Int. Cl.: A61K 47/48, A61K 9/16, A23J 3/00, A23L 1/302, A23L 1/303, A23L 1/305, A23K 1/16

(54) **Stabile pulverförmige Vitamin- und Carotinoid-Zubereitungen und Vefahren zu deren Herstellung**
Stable powdery vitamin and carotenoide containing compositions and process to prepare them
Préparations pulvérulentes à la vitamin et le caroténoides et procédé de préparation

(30) Priorität: 24.08.1998 DE 19838189
(43) Veröffentlichungstag der Anmeldung: 01.03.2000
(73) Patentinhaber: N-Zyme BioTec GmbH, 64287 Darmstadt (DE)
(72) Erfinder: Bewert, Wolfgang, Dr., 67227 Frankenthal (DE); Betz, Roland, Dr., 67150 Niederkirchen (DE); Lüddecke, Erik, Dr., 67112 Mutterstadt (DE); Friedrich, Thomas, Dr., 64283 Darmstadt (DE)
(74) Vertreter: Werner, Hans-Karsten, Dr.Dipl.-Chem.

(56) Entgegenhaltungen:
- EP-A- 0 618 001
- FR-A- 2 659 352
- US-A- 4 670 247
- PATENT ABSTRACTS OF JAPAN vol. 018, no. 367 (C-1223), 11. Juli 1994 (1994-07-11) & JP 06 098743 A (NIPPI ZERACHIN KOGYO KK;OTHERS: 01), 12. April 1994 (1994-04-12)
- PATENT ABSTRACTS OF JAPAN vol. 007, no. 100 (C-164), 28. April 1983 (1983-04-28) & JP 58 028234 A (YUKIJIRUSHI NIYUUGIYOU KK), 19. Februar 1983 (1983-02-19)

## Beschreibung

Die vorliegende Erfindung betrifft Wirkstoffzubereitungen, bei denen ein oder mehrere Wirkstoffe in eine Proteinmatrix eingebettet sind, wobei die Proteine mit Transglutaminase quervernetzt sind. Die Wirkstoffzubereitungen liegen z.B. als stabile Trockenpulver vor. Die Erfindung betrifft außerdem Nahrungs- und Futtermittel enthaltend solche Zubereitungen, Verfahren zur Herstellung der Trockenpulver und spezielle Verwendungen der quervernetzten Proteine. Im Besonderen betrifft die Erfindung Trockenpulver, die Vitamine, Lebensmittel- und Futtermittelzusatzstoffe, wie z.B. Carotinoide enthalten. Außerdem können noch verschiedene Zusatzstoffe eingebettet sein.

Pulverförmige Vitamin- und Carotinoid-Präparate sind allgemein bekannt und werden in der pharmazeutischen Industrie sowie in der Nahrungs- und Futtermittelindustrie in großem Umfang verwendet. So sind in der Literatur viele Verfahren zur Herstellung geeigneter Präparate beschrieben.

Zur Herstellung von pulverförmigen Zubereitungen, in denen oxidationsempfindliche Stoffe wie öllösliche Vitamine oder Carotinoide gegen oxidative Einflüsse geschützt werden sollen, werden verschiedene Herstellverfahren, insbesondere Sprühverfahren beschrieben.

In der deutschen Patentschrift 1 035319 wird beschrieben, wie eine Dispersion eines öligen Vitamins in einen hohen Überschuß von pulverförmiger Stärke mit einem geringen Feuchtegehalt (unter 8 %) versprüht wird. Durch das trockene Stärkepulver wird den versprühten Partikeln Wasser entzogen. Hierdurch erstarren sie, wobei eine große Menge der Stärke an der Oberfläche der Partikel haften bleibt. Außerdem muß der überschüssige Stärkeanteil abgetrennt und anschließend wieder dem Prozeß zugeführt werden.

In der schweizerischen Patentschrift 488 455 wird dargelegt, daß als Puderungsmittel ein Gemisch aus anorganischen Substanzen eingesetzt wird, das aus wasserabweisenden und wasserabsorbierenden Substanzen besteht. Hierdurch soll die Explosionsgefahr, die durch die trockene, feinverteilte Stärke besteht, vermieden werden.

Aus der schweizerischen Patentschrift 389 505 ist bekannt, daß die Dispersion des Wirkstoffs in ein gekühltes, gasförmiges Medium versprüht wird, in dem die versprühten Partikel durch Abkühlung erstarren. Für diesen Prozeß werden Fallhöhen von bis zu 15 m benötigt, dazu müssen die Temperaturen deutlich unter Raumtemperatur gehalten werden.

Eine weitere Methode zur Verfestigung der versprühten Partikel kann auch durch Auffangen in einem Pulver, das aus Metallsalzen höherer Fettsäuren besteht, erfolgen. Dieses Verfahren wird in der schweizerischen Patentschrift 431 252 beschrieben.

Eine alternative Methode zur Herstellung von wirkstoffhaltigen, stabilen Zubereitungen wird in der europäischen Patentschrift 0 618 001 beschrieben. Hier erfolgt die Herstellung der kugelförmigen Partikel, die eine Einbettung des Wirkstoffs in einem Gemisch aus verschiedenen Trägersubstanzen darstellen, indem man zunächst durch kontrollierte Teilung Kügelchen aus einer primären Öl-in-Wasser-Emulsion bildet, die aus der Zugabe von Wirkstoffen, ölförmigen Stoffen, Proteinen und Wasser zum einem nicht mit Wasser mischbaren Lösungsmittel besteht, und anschließend die erhaltenen Kügelchen abtrennt. Für die Herstellung der Kügelchen ist ein spezielles Mischsystem erforderlich. Die hierbei erhaltenen Partikel werden anschließend mit einem Aldehyd, beispielsweise Acetaldehyd, Glutaraldehyd oder Glyoxal nachbehandelt, wodurch eine chemische Vernetzung, die sich auch in der Wasserunlöslichkeit des erhaltenen Materials ausdrückt, und damit eine zusätzliche Stabilisierung des Wirkstoffs erreicht wird.

In der amerikanischen Patentschrift 4 670 247 wird eine weitere Methode zur Herstellung von vernetzten Partikeln beschrieben. Hierzu wird zunächst eine Emulsion, die im wesentlichen aus einem öllöslichen Vitamin, einem Schutzkolloid wie z.B. Gelatine und einem reduzierenden Zucker besteht, durch einen Sprüh- und Trocknungsprozeß in pulverförmige Partikel überführt. Diese Partikel werden anschließend in einem thermischen Prozeß bei Temperaturen zwischen 105 und 180°C behandelt. Durch eine Maillard-Reaktion zwischen den Aminogruppen der Proteine und den Oxo-Gruppen der reduzierenden Zucker wird eine Wasserunlöslichkeit der Trockenpulverpartikel erzielt, die durch eine Vernetzung der Matrixbestandteile erreicht wird.

In EP 782883 werden eßbare Mikrokapseln beschrieben, die eine Kapselwand enthalten, die durch Aussalzen des Proteins mit einem eßbaren Salz und Vernetzung der Kapselwand mit Transglutaminase hergestellt werden.

Wenn oxidationsempfindliche Verbindungen, in diesem Falle insbesondere fettlösliche Vitamine und Carotinoide, mit Luft in Kontakt kommen, erfolgt eine Umsetzung mit Sauerstoff, die zu einem Wirkstoffverlust durch Umsetzung zu ungewünschten Verbindungen führt. Um diese Oxidation zu verhindern, kann man zum Beispiel bestimmte Zusätze den Zubereitungen hinzufügen, die durch Umsetzung mit reaktiven Gruppen der Proteine diese wiederum weniger durchlässig für Sauerstoff machen und dadurch den Wirkstoffen einen stabilisierenden Schutz verleihen. Dies kann beispielsweise dadurch geschehen, daß durch Umsetzung von Proteinen mit reduzierenden Zuckern im Sinne einer Maillard-Reaktion die Löslichkeit der Proteine in Wasser verhindert wird. Weiterhin kann durch Umsetzung der Proteine mit Aldehyden eine Vernetzung erreicht werden, die ebenfalls der Trägermatrix eine erhöhte Stabilität verleiht.

Diese Verfahren zeigen jedoch bestimmte Nachteile, die es wünschenswert erscheinen lassen, nach verbesserten Stabilisierungsverfahren zu suchen. So bedeutet die Anwendung einer Maillard-Reaktion zwischen einem Protein und reduzierenden Zuckern in jedem Falle eine thermische Belastung und damit zumindest in einem geringen Maße einen Abbau des Wirkstoffs. Außerdem neigen die Produkte zu einer bräunlichen Färbung.

Die Verwendung von Aldehyden als Vernetzungsmittel ist mit dem schwerwiegenden Nachteil verbunden, daß hier hochreaktive und gesundheitlich höchst bedenkliche Zusatzstoffe eingesetzt werden. Die Produkte, die nach diesen Verfahren hergestellt werden, finden beim Verbraucher nur bedingt unumschränkte Akzeptanz.

Dagegen bedeutet die Verwendung einer enzymatischen Vernetzung als stabilisierendes Prinzip sowohl die Vermeidung von thermischen Prozessen als auch eine in jeder Hinsicht unbedenkliche Hinzufügung eines Stoffes, der ein natürlicher Bestandteil jeder Nahrungskette ist. Die Produkte weisen außerdem keine bräunliche Färbung auf.

Das erfindungsgemäße Verfahren zur Herstellung von Wirkstoffzubereitungen umfaßt die Schritte:
a) Durchmischen einer wäßrige Lösung aus vernetzbaren Protein, Transglutaminase und Wirkstoff
b) Versprühen,
wobei das Gewichtsverhältnis zwischen Wirkstoff und Protein zwischen 1 zu 10 und 4 zu 1 liegt.

Bevorzugte Gewichtsverhältnisse zwischen Wirkstoff und Protein liegen zwischen 1 zu 4 und 2 zu 1, besonders bevorzugt zwischen 1 zu 3 und 1 zu 1.

Bevorzugte vernetzbare Proteine sind Gelatine, Kasein, SojaProtein, Mais-Protein und Kollagen.

Bevorzugt sind erfindungsgemäße Verfahren, bei denen eine wirkstoffhaltige Emulsion oder Dispersion in eine mit hydrophober Kieselsäure beladene Inertgas-Atmosphäre versprüht wird.

Außerdem sind Verfahren bevorzugt, bei denen nach dem Versprühen getrocknet wird bis zu einer Restfeuchte unter 10 Gew.-%, bevorzugt unter 6 Gew.-%.

Die Temperatur des erfindungsgemäßen Verfahrens wird im Wesentlichen unter 80°C, bevorzugt unter 60°C, gehalten.

Die Erfindung betrifft außerdem Wirkstoffzubereitungen erhältlich nach einem erfindungsgemäßen Verfahren, und solche, die als zusätzliches Merkmal den 0,025-fachen bis 4-fachen Gewichtsanteil bezüglich Wirkstoff an Trennmittel oder Trennmittelgemischen enthalten, sowie Lebensmittel oder Futtermittel enthaltend eine solche Wirkstoffzubereitung.

Bevorzugte Trennmittel sind hydrophobe Kieselsäure, Maisstärke, durch chemische Behandlung hydrophobierte Maisstärke, Metallsalze höherer Fettsäuren und andere pflanzliche Stärken.

Im Fall von hydrophober Kieselsäure liegt der Anteil bezüglich Wirkstoff bevorzugt in einem Bereich zwischen 0,025 und 0,4, besonders bevorzugt zwischen 0,05 und 0,2. Bei Maisstärke liegt das entsprechende Verhältnis bevorzugt zwischen 0,25 und 2, besonders bevorzugt zwischen 0,5 und 1,5.

Die erfindungsgemäßen Wirkstoffzubereitungen sind erhältlich durch Herstellung einer Dispersion enthaltend im wesentlichen diese Wirkstoffe, ein Protein und weitere Träger- und Füllstoffe z.B. aus der Gruppe der Kohlenhydrate und/oder natürliche oder chemisch modifizierte Stärken. Sie kann noch weitere Zusatzstoffe wie Stabilisatoren oder Emulgierhilfsmittel enthalten. Außerdem enthält sie ein Enzym, das in der Lage ist, auf verschiedene Weise Proteinmoleküle miteinander zu verknüpfen. Die hierdurch erreichte Vernetzung verleiht dem Protein und damit auch der Matrix, in dem die Wirkstoffe eingebettet sind, eine verminderte Wasserlöslichkeit und somit eine erhöhte Stabilität.

Die erhaltenen Wirkstoffzubereitungen sind im wesentlichen homogen bezüglich der Verteilung von vernetzter Matrix.

Bevorzugte vernetzbare Proteine sind Gelatine, z.B. Knochengelatine, Rindergelatine, Fischgelatine, Milchproteine, wie z.B. Kasein, Sojaproteine, Maisproteine und Kollagene, besonders bevorzugte Proteine sind Milchproteine und Sojaproteine. Die erfindungsgemäßen vernetzenden Enzyme sind Transglutaminasen, besonders solche, die aus Mikroorganismen gewonnen werden, insbesondere Transglutaminasen mikrobiellen Ursprungs.

Bevorzugte Wirkstoffe sind Vitamine, Lebensmittel- und Futtermittelzusatzstoffe. Insbesondere sind hydrophobe Wirkstoffe bevorzugt, besonders bevorzugt solche die leicht oxidierbar sind. Solche sind die Vitamine der Gruppen A, D, E und K. Bevorzugte Lebensmittel- und Futtermittelzusatzstoffe sind Carotine und Carotinoide wie β-Carotin und wie z.B.: Astaxanthin, Astacin, Apo-8'-carotinsäureethylester, Citranaxanthin, Canthaxanthin, Zeaxanthin, Apo-8'-carotinal, Lutein, Capsanthin, Lycopin und deren Mischungen.

Die Emulsion wird unter Verwendung von hydrophoben Trennmitteln wie hydrophobe Kieselsäure, natürliche Stärke, wie z.B. Maisstärke, hydrophobierte Stärkederivate, wie z.B. hydrophobierte Maisstärke, Salzen von langkettigen Fettsäuren oder Gemischen aus diesen Stoffen versprüht. Das Trennmittel kann aber auch in der Sprühkammer z.B. als hydrophobe Kieselsäurepartikel in Inertgas (z.B. Stickstoff) vorgelegt werden. Anschließend erfolgt die Trocknung der versprühten Partikel gegebenenfalls nach Abtrennung der Trennmittel, gegebenenfalls unter leichter Erwärmung bis 80°C, bevorzugt bis 60°C, besonders bevorzugt bei Raumtemperatur, durch Behandlung in einem Luft- oder Schutzgasstrom.

### Beispiel 1

In 360 g Wasser wurden 81,8 g Gelatine (Typ A 100 Bloom) und 50,7 g Isosweet (Fa. Amylum) dispergiert und durch 30-minütiges Rühren bei 60°C in Lösung gebracht. Anschließend wurden 62,6 g Maisstärke hinzu gegeben, wobei weiter gerührt wurde, bis alle Komponenten gleichmäßig dispergiert waren. Danach erfolgte die Zugabe von 62,9 g Vitamin A-Acetat, das durch Zugabe von Ethoxyquin (100 mg/Mio I.E.) und BHT (4,5 mg/Mio I.E.) stabilisiert wurde. Das Vitamin A wurde mit Hilfe eines Hochleistungsrührers in die wäßrige Phase einemulgiert. Schließlich wurde die erhaltene Emulsion durch Zugabe von 10%iger wäßriger Natronlauge auf einen pH-Wert von 8,0 eingestellt und mit 20 ml einer wäßrigen Lösung von 200 Units bakterieller Transglutaminase versetzt.

Die erhaltene Emulsion wurde danach bei 55°C über eine Einstoffdüse bei einem Druck von 4,2 bar in eine mit hydrophober Kieselsäure beladene Stickstoffatmosphäre versprüht. Das erhaltene Produkt wurde anschließend auf einem Wirbelbett-Trockner im Stickstoffstrom bei Raumtemperatur innerhalb 15 Std. auf eine Restfeuchte von 4,2 % getrocknet.

### Beispiel 2

In Abänderung des vorhergehenden Beispiels wurde ein Trockenpulver wie beschrieben, jedoch ohne Zusatz von Transglutaminase, hergestellt. Dieses Produkt wies nach dem Trocknen einen Restfeuchtegehalt von 3,9 % auf.

### Beispiel 3

Ein Teil des Trockenpulvers aus Beispiel 2 wurde durch thermische Behandlung in einem rotierenden Kolben in einem Ölbad bei 120°C 20 Min lang getempert, wobei durch Maillard-Reaktion eine bräunliche Verfärbung eintrat. Die Restfeuchte des Produkts sank bei diesem Prozeß auf einen Gehalt von 1,9 %.

### Beispiel 4

Eine Astaxanthin-Formulierung wird nach dem in EP 0 065 193 im Detail beschriebenen Verfahren hergestellt.

30 g Astaxanthin werden in 240 g Isopropanol gemeinsam mit 1,1 g Ascorbylpalmitat und 6,4 g Ethoxyquin suspendiert und bei Einstellung des Druckbegrenzungsventils aus 30 bar mit 370 g Isopropanol in einer Mischkammer kontinuierlich gemischt. Bei einer Dosiergeschwindigkeit von 6 l/h auf der Suspensionsseite und von 9 l/h auf der Lösungsmittelseite wird in der Mischkammer eine Mischungstemperatur von 173°C eingestellt. Nach einer Verweilzeit von 0,3 Sekunden wird die molekular-disperse Lösung in einer weiteren Mischkammer mit einer auf pH 9,5 eingestellten Lösung von 38,6 g Gelatine und 105 g Dextrose in 4000 g Wasser bei einer Durchsatzgeschwindigkeit von 80 l/h gemischt. Man erhält eine kolloid-disperse Wirkstoffsuspension. Die Teilchengrößenanalyse liefert einen Mittelwert von 0,15 µm bei einer Verteilungsbreite von ± 31 %.

An einem Dünnschichtverdampfer wird das Mikronisat bis auf einen Feststoffgehalt von ca. 25 % aufkonzentriert. Das aufkonzentrierte Produkt wird nun bei 60°C aufgeschmolzen, 50 ml einer wäßrigen Lösung von 250 Units bakterieller Transglutaminase werden zugesetzt und mit einem Blattrührer in einem Vierhalskolben untergerührt.

Die fertige Dispersion wird in einen Autoklaven gefüllt und mit einer Düse in einen Behälter, der Sipernat D17 als Sprühhilfsmittel in Luft dispergiert enthält, versprüht. Das so hergestellte Trockenpulver wird auf einem Wirbelbett-Trockner bei Raumtemperatur innerhalb 20 h auf einen Feuchtegehalt von < 6 % getrocknet.

### Beispiel 5

Aus den erhaltenen Trockenpulvern wurde die Fraktion mit der Teilchengröße 250 bis 355 µm herausgesiebt und einer Stabilitätsprüfung in einen Standardprämix unterzogen. Hierzu wurden ca. 100 mg der Prüfmuster in Präperategläschen eingewogen (je Muster und Prüfzeitpunkt 4 Einwägungen), mit 4 g Prämixmischung, bestehend aus 60 % Weizengrießkleie, 30 % 50 %igem Cholinchlorid auf Kieselsäure und 10 % Spurenelementmischung, bestehend aus 37,43 % CuSO₄ × 5 H₂O; 46,78 % FeSO₄ × 7 H₂O; 11,79 % ZnO; 3,61 % MnO und 0,39 % CoCO₃ versetzt und anschließend sorgfältig mit der Hand gemischt.

Die Prüfmuster werden in einem Klimaschrank bei konstanter Temperatur und Feuchte (40°C und 70 % rel. Feuchte) 6 Wochen lang offen gelagert. Zu Beginn der Lagerung, nach 4 und 6 Wochen werden die 4 für den jeweiligen Prüfzeitpunkt vorbereiteten Prüfmuster entnommen und auf den verbleibenden Restgehalt an Vitamin A Wirkstoff hin überprüft.

Die Prüfungsergebnisse sind in der folgenden Tabelle dargestellt:

### Vitamin A-Retention (%)

### Versuchsprodukt

| Trockenpulver | Start (I.E./g) | nach 4 Wochen | nach 6 Wochen |
|---|---|---|---|
| 1. mit Zusatz von Transglutaminase | 494.884 | 86,7 | 79,5 |
| 2. ohne Transglutaminase | 520.924 | 85,1 | 76,6 |
| 3. wie 2., thermisch behandelt | 526.144 | 87,4 | 80,7 |

### Beispiel 6

Gemäß Beispiel 1 wurde mit 420 g Wasser, 102,3 g Gelatine 100 Bloom A, 63,4 g Isosweet, 76,5 g Vitamin A-Acetat (2,16 mio I.E./g; stabilisiert mit 100 mg Ethoxyquin/Mio I.E. und 14,5 mg BHT/Mio I.E.) und 80,6 g Maisstärke eine Emulsion hergestellt, die anschließend mit 10%iger wäßriger Natronlauge auf einen pH-Wert von 8,0 eingestellt wurde. Diese Emulsion wurde in drei gleiche Portionen aufgeteilt und wie folgt behandelt:
A: kein Zusatz
B: Zusatz von 2,05 g einer 1%igen Zubereitung von Transglutaminase ("TG") in einem Polysaccharid
C: Zusatz von 5,11 g einer 1%igen Zubereitung von Transglutaminase ("TG") in einem Polysaccharid

Die Emulsion wurde gemäß Beispiel 1 in eine mit hydrophober Kieselsäure beladene Stickstoffatmosphäre versprüht.

Ansatz A wurde im Stickstoffstrom bei Raumtemperatur auf eine Restfeuchte von 3,1 % getrocknet. Hiervon wurde ein Teil zusätzlich bei 120°C 20 min getempert.

Ansatz B wurde sowohl bei Raumtemperatur getrocknet als auch nach einer Lagerung von 15 Std. bei Raumtemperatur ebenfalls im Wirbelbett getrocknet.

Ansatz C wurde entsprechend Ansatz B behandelt.

Die erhaltenen Produkte wurden gemäß Beispiel 5 einer Stabilitätsprüfung unterzogen. Die ermittelten Daten werden in nachstehender Tabelle zusammengestellt.

| Produkt | Behandlung | Vitamin-A-Gehalt (I.E.-/g) | Restefeuchte (%) | Retention nach 4 Wochen (%) | Retention nach 6 Wochen (%) |
|---|---|---|---|---|---|
| Ohne Zusatz | Wirbelbett-Tr. (= Trocknung) | 548.700 | 3,1 | 53,9 | 29,1 |
| Ohne Zusatz | Wirbelbett-Tr. + therm. Vernetzung | 563.300 | 1,5 | 63,8 | 45,9 |
| 2,05 g TG | Wirbelbett-Tr. | 543.600 | 3,9 | 56,2 | 34,5 |
| 2,05 g TG | 15 Std. RT + wirbelbett-Tr. | 541.900 | 3,4 | 54,8 | 32,4 |
| 5,11 g TG | Wirbelbett-Tr. | 540.000 | 3,2 | 62,6 | 35,5 |
| 5,11 g TG | 15 Std. RT + Wirbelbett-Tr. | 544.600 | 3,6 | 64,4 | 39,7 |

| Produkt | Behandlung | Diffusionsgeschwindigkeit (usec, Tau) | Intensität (kcps) |
|---|---|---|---|
| Ohne Zusatz | wirbelbett-Tr. | 596,3 | 559,3 |
| | | 581,2 | 579,0 |
| | | 552,2 | 543,3 |
| Ohne Zusatz | Wirbelbett-Tr. + therm. Vernetzung | | |
| 2,05 g TG | Wirbelbett-Tr. | 510,7 | 545,4 |
| | | 514,6 | 536,4 |
| | | 496,9 | 536,4 |
| 2,05 TG | 15 Std. RT + Wirbelbett-Tr. | | |
| 5,11 g TG | Wirbelbett-Tr. | 544,0 | 518,3 |
| | | 574,4 | 539,1 |
| | | 549.5 | 542,3 |
| 5,11 g TG | 15 Std. RT + Wirbelbett-Tr. | | |

Gelatine ist ein Gemisch verschieden großer Proteinmoleküle. Alle Moleküle werden durch den Fluoreszenzfarbstoff markiert. Bei starker Quervernetzung gelingt es nur den kleinsten Molekülen im Beobachtungszeitraum, das Netzwerk der quervernetzten Gelatine zu verlassen. Darüber hinaus haben kleinere Moleküle eine geringere Wahrscheinlichkeit, von einer Quervernetzung direkt betroffen zu sein. Beobachtet man durch FCS die Molekülgröße im Überstand der durch Transglutaminase stark quervernetzten Gelatine, so findet man eine hohe Diffusionsgeschwindigkeit (Tau in µ Sekunden ist kleiner als in der Kontrolle) - typisch für kleine Molekülgrößen. Auch ist zu beobachten, daß die Intensität (I, kcps) weitgehend vergleichbar zwischen allen Proben ist.

### Beispiel 7

In einem weiteren Versuch wurde eine Astaxanthin-Dispersion unter Zusatz unterschiedlicher Mengen Transglutaminase gemäß Beispiel 4 zu Trockenpulvern verarbeitet.

| Zusatz | Behandlung | WirkstoffGehalt t (%) | Retention nach 6 Wochen (%) |
|---|---|---|---|
| Ohne | Bei RT getrocknet | 11,4 | 67 |
| 0,01 % TG | Bei RT getrocknet | 11,5 | 80 |
| 0,05 % TG | Bei RT getrocknet | 11,2 | 75 |
| RT = Raumtemperatur ∼ 23°C | | | |

## Patentansprüche

1. Verfahren zur Herstellung von Wirkstoffzubereitungen umfassend die Schritte
a) Durchmischen einer wäßrige Lösung aus vernetzbarem Protein, Transglutaminase und Wirkstoff
b) Versprühen,
wobei das Gewichtsverhältnis zwischen Wirkstoff und Protein zwischen 1 zu 10 und 4 zu 1 liegt.

2. Verfahren nach Anspruch 1, wobei Wirkstoffe Vitamine, Lebensmittelzusatzstoffe oder Futtermittelzusatzstoffe sind.

3. Verfahren nach einem der Ansprüche 1 oder 2, wobei die Wirkstoffe hydrophob sind.

4. Verfahren nach einem der Ansprüche 1 bis 3, wobei das vernetzbare Protein ausgewählt aus der Liste Gelatine, Kasein, Sojaprotein, Maisprotein und Kollagen ist.

5. Verfahren nach einem der Ansprüche 1 bis 4, wobei eine Transglutaminase gewonnen aus Mikroorganismen verwendet wird.

6. Verfahren nach einem der Ansprüche 1 bis 5, wobei in eine mit hydrophober Kieselsäure, Maisstärke oder hydrophobierte Maisstärke beladene Inertgas Atmosphäre versprüht wird.

7. Verfahren nach einem der Ansprüche 1 bis 6, wobei nach dem Versprühen getrocknet wird bis zu einer Restfeuchte unter 10 Gew.-%.

8. Verfahren nach einem der Ansprüche 1 bis 7, wobei die Temperatur der Wirkstoffzubereitung im Wesentlichen unter 80°C gehalten wird.

9. Wirkstoffzubereitung erhältlich nach einem Verfahren gemäß einem der Ansprüche 1 bis 8.

10. Wirkstoffzubereitung nach Anspruch 9, wobei die Wirkstoffe ausgewählt sind aus der Gruppe:
Carotinoide
Vitamin A
Vitamin E
Vitamin D₃

11. Wirkstoffzubereitung nach einem der Ansprüche 9 oder 10, die den 0,025-fachen bis 4-fachen Gewichtsanteil bezüglich Wirkstoff an Trennmittel oder Trennmittelgemischen enthält.

12. Lebensmittel oder Futtermittel, enthaltend eine Wirkstoffzubereitung nach einem der Ansprüche 9 bis 11.

## Claims

1. A process for preparing formulations of active substances, comprising the steps of
a) mixing an aqueous solution of cross-linkable protein, transglutaminase and an active substance;
b) spraying the solution;
wherein the weight ratio of active substance to protein is between 1 to 10 and 4 to 1.

2. The process according to claim 1, wherein said active substances are vitamins, food additives or fodder additives.

3. The process according to either of claims 1 or 2, wherein said active substances are hydrophobic.

4. The process according to any of claims 1 to 3, wherein said cross-linkable protein is selected from the group consisting of gelatin, casein, soybean protein, maize protein and collagen.

5. The process according to any of claims 1 to 4, wherein a transglutaminase obtained from microorganisms is used.

6. The process according to any of claims 1 to 5, wherein said spraying is performed into an inert gas atmosphere loaded with hydrophobic silica, maize starch or hydrophobized maize starch.

7. The process according to any of claims 1 to 6, wherein said spraying is followed by drying until the residual moisture is below 10% by weight.

8. The process according to any of claims 1 to 7, wherein the temperature of said formulation of active substances is essentially maintained below 80 °C.

9. A formulation of active substances obtainable by the process according to any of claims 1 to 8.

10. The formulation of active substances according to claim 9, wherein said active substances are selected from the group of:
carotinoids;
vitamin A;
vitamin E;
vitamin D₃.

11. The formulation of active substances according to either of claims 9 or 10, containing a parting agent or mixture of parting agents at a weight proportion which is 0.025 to 4 times the weight of the active substance.

12. A food or fodder containing a formulation of active substances according to any of claims 9 to 11.

## Revendications

1. Procédé de préparation de compositions de principes actifs, comprenant les étapes de
a) mélange intime d'une solution aqueuse d'une protéine réticulable, d'une transglutaminase et d'un principe actif,
b) pulvérisation,
le rapport en poids entre le principe actif et la protéine étant compris entre 1 à 10 et 4 à 1.

2. Procédé selon la revendication 1, dans lequel les principes actifs sont des vitamines, des additifs pour produits alimentaires ou des additifs pour aliments pour animaux.

3. Procédé selon la revendication 1 ou 2, dans lequel les principes actifs sont hydrophobes.

4. Procédé selon l'une des revendications 1 à 3, dans lequel on choisit la protéine réticulable dans la liste comprenant : la gélatine, la caséine, la protéine de soja, la protéine de mais et le collagène.

5. Procédé selon l'une des revendications 1 à 4, dans lequel on utilise une transglutaminase obtenue à partir de micro-organismes.

6. Procédé selon l'une des revendications 1 à 5, dans lequel on effectue la pulvérisation dans une atmosphère de gaz inerte chargée d'acide silicique hydrophobe, d'amidon de maïs ou d'amidon de maïs rendu hydrophobe.

7. Procédé selon l'une des revendications 1 à 6, dans lequel, après la pulvérisation, on effectue un séchage jusqu'à l'obtention d'une humidité résiduelle inférieure à 10 % en poids.

8. Procédé selon l'une des revendications 1 à 7, dans lequel on maintient la température de la composition de principes actifs sensiblement au-dessous de 80°C.

9. Composition de principes actifs pouvant être obtenue conformément à un procédé selon l'une des revendications 1 à 8.

10. Composition de principes actifs selon la revendication 9, dans laquelle on choisit les principes actifs dans l'ensemble comprenant :
les caroténoïdes
la vitamine A
la vitamine E et
la vitamine D₃.

11. Composition de principes actifs selon la revendication 9 ou 10, contenant une proportion en poids d'agents de séparation ou de mélanges d'agents de séparation de 0,025 fois à 4 fois par rapport au principe actif.

12. Produit alimentaire ou aliment pour animaux contenant une composition de principes actifs selon l'une des revendications 9 à 11.
